# EUROPEAN PATENT APPLICATION

(11) **EP 2 438 893 A1**
(43) Date of publication of application: **11.04.2012**
(21) Application number: 10783460.8
(22) Date of filing: 04.06.2010
(51) Int. Cl.: A61F 13/15, A61F 13/49, A61F 13/53

(54) **ABSORBENT GOODS AND METHOD FOR MANUFACTURING ABSORBENT GOODS**

(30) Priority: 05.06.2009 JP 2009136437
(71) Applicant: Unicharm Corporation, Ehime 799-0111 (JP)
(72) Inventor: MUKAI, Hirotomo, Kanonji-shi Kagawa 769-1602 (JP); TSUJI, Tomoko, Kanonji-shi Kagawa 769-1602 (JP); TAKAHASHI, Natsuko, Kanonji-shi Kagawa 769-1602 (JP); WATABE, Yoshihisa, Kanonji-shi Kagawa 769-1602 (JP)
(74) Representative: Peter, Julian
(86) International application number: PCT/JP2010/059516
(87) International publication number: WO 2010/140678

(57) **Abstract**

An absorbent article 1 according to the present invention includes leg elastic bodies 7A and a central elastic body 60. An absorber 40 has a bent part 43 formed along a longitudinal direction L. The leg elastic bodies 7A continuously extend from one rear waistline edge 6 to the other rear waistline edge 6 in a state of being stretched in a width direction W in a rear waistline region S2. The central elastic body 60 is provided in a central portion C in the width direction W in a state of being stretched along the longitudinal direction L, while overlapping with any one of a front waistline region S1 and the rear waistline region S2 in a thickness direction T. At least a part of the central elastic body 60 intersects with the leg elastic bodies 7A while overlapping with the bent part 43 in the thickness direction T.

## Description

### [Technical Field]

The present invention relates to an absorbent article having a central elastic body provided in a central portion in a width direction of the absorbent article, the central elastic body being stretchable in a longitudinal direction of the absorbent article, and also relates to a method for manufacturing the absorbent article.

### [Background Art]

There has heretofore been known an absorbent article, such as a disposable diaper, in which a long and narrow central elastic body made of an urethane film, a stretchable nonwoven fabric or the like is provided in a stretched state in a central portion in a width direction of the absorbent article, the central elastic body being stretchable in a longitudinal direction of the absorbent article (in a front-back direction when in use) (e.g., see Patent Document 1).

A crotch region of an absorbent article, which corresponds to the crotch of a wearer, is sandwiched between thighs of the wearer. Thus, in the crotch region, multiple wrinkles occur along a longitudinal direction of the absorbent article due to irregular deformation.

In the above absorbent article, irregular deformation of the absorbent article is controlled by a central elastic body causing a central portion of the crotch region to contract in the longitudinal direction of the absorbent article. Thus, such wrinkles that inhibit absorption of bodily waste are prevented from occurring.

### [Prior Art Document]

### [Patent Document]

[Patent Document 1] Japanese Patent Application Publication No. Hei 11-188056 (pp. 2-3, Figs. 1 and 3)

### [Summary of Invention]

However, the conventional absorbent article described above has the following problems. Specifically, one end of the central elastic body, which is positioned on the stomach side of the wearer during wearing the absorbent article, and the other end of the central elastic body, which is positioned on the back side thereof, contract in a direction of reducing a distance between the ends toward the center of the crotch region. Thus, the stomach-side portion and back-side portion of the absorbent article are likely to come down toward the crotch of the wearer. Therefore, an absorber of the absorbent article is likely to separate from the skin of the wearer, causing the absorbent article to be less comfortably worn by the wearer. Moreover, when the absorber separates from the skin of the wearer, the bodily waste is not absorbed by the absorber immediately after being excreted. This results in leakage from the absorbent article.

An absorbent article of an aspect include: a topsheet which comes into contact with the skin of a wearer; an outer sheet positioned on an outer side during wearing; an absorber provided between the topsheet and the outer sheet; a front waistline region corresponding to a front waistline of the wearer; a rear waistline region corresponding to a rear waistline of the wearer; a first elastic body provided to be stretchable along a width direction of the absorbent article; and a second elastic body provided to be stretchable along a longitude direction of the absorbent article. The front waistline region and the rear waistline region each has a pair of edges positioned at outer ends in the width direction of the absorbent article. The absorber has a bent part formed along the longitude direction of the absorbent article. The first elastic body continuously extends from one edge to the other edge in at least one of the front waistline region and the rear waistline region. The second elastic body is provided in a central portion in the width direction of the absorbent article in a state of being stretched in the longitude direction of the absorbent article, and overlaps with any one of the front waistline region and the rear waistline region in a thickness direction of the absorbent article. At least a part of the second elastic body intersects with the first elastic body while overlapping with the bent part in the thickness direction of the absorbent article.

### [Brief Description of Drawings]

[Fig. 1] Fig. 1 is an exploded perspective view showing an absorbent article 1 according to a first embodiment.
[Fig. 2] Fig. 2 is a plan view showing the absorbent article 1 according to the first embodiment.
[Fig. 3] Fig. 3 is a cross-sectional view (cross-sectional view taken along the line A-A in Fig. 2) showing the absorbent article 1 according to the first embodiment.
[Fig. 4] Fig. 4 is a cross-sectional view (cross-sectional view taken along the line B-B in Fig. 2) showing the absorbent article 1 according to the first embodiment.
[Fig. 5] Fig. 5 is a plan view showing a first crotch member 90A according to the first embodiment.
[Fig. 6] Fig. 6 is a view for illustrating a method for manufacturing an absorbent article according to the first embodiment.
[Fig. 7] Fig. 7 is an exploded perspective view (No. 1) showing an absorbent article 1A according to modified example 1.
[Fig. 8] Fig. 8 is an exploded perspective view (No. 2) showing an absorbent article 1B according to modified example 1.
[Fig. 9] Fig. 9 is a view for illustrating a method for manufacturing an absorbent article according to modified example 1.
[Fig. 10] Fig. 10 is a plan view showing a first crotch member 90A of an absorbent article 1C according to modified example 2.
[Fig. 11] Fig. 11 is an exploded perspective view (No. 1) showing an absorbent article 1D according to a second embodiment.
[Fig. 12] Fig. 12 is a plan view showing the absorbent article 1D according to the second embodiment.
[Fig. 13] Fig. 13 is an exploded perspective view (No. 2) showing an absorbent article IE according to the second embodiment.
[Fig. 14] Fig. 14 is an exploded perspective view showing an absorbent article 1F according to a third embodiment.
[Fig. 15] Fig. 15 is a cross-sectional view showing only the absorbent article 1F according to the third embodiment.
[Fig. 16] Fig. 16 is a view for illustrating a method for manufacturing an absorbent article according to the third embodiment.
[Fig. 17] Fig. 17 is an exploded perspective view showing an absorbent article 1G according to a fourth embodiment.

### [Description of Embodiments]

With reference to the drawings, an absorbent article and a method for manufacturing the absorbent article according to the present invention will be described below. Specifically, first to fourth embodiments and other embodiments will be described.

In the following description of the drawings, the same or similar parts are denoted by the same or similar reference numerals. However, it should be noted that the drawings are schematic and their dimensional ratios and the like are different from actual ones.

Therefore, specific dimensions and the like should be judged in consideration of the following description. In addition, it goes without saying that the drawings include parts whose dimensional relationship and ratios differ among the drawings.

### [First Embodiment]

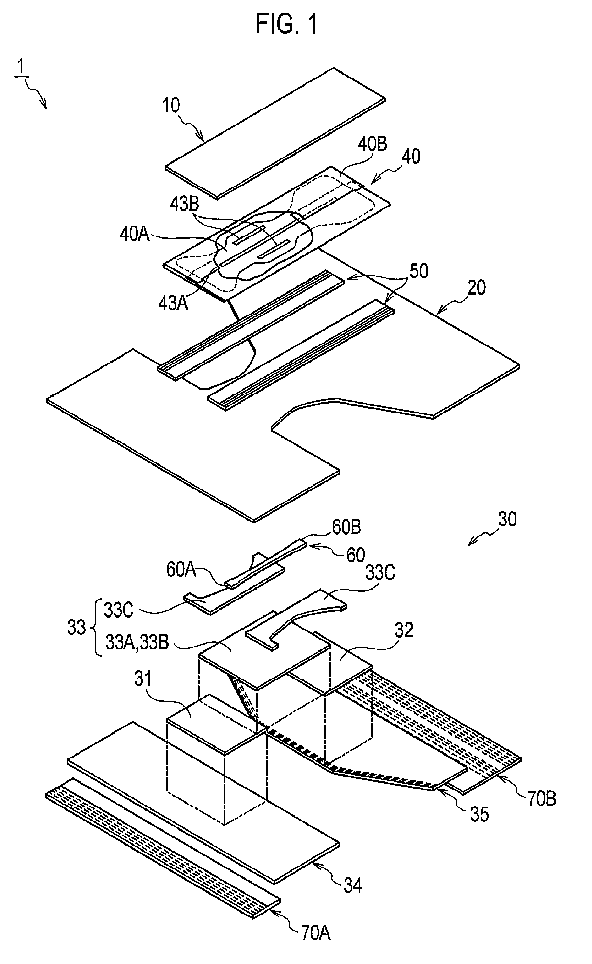

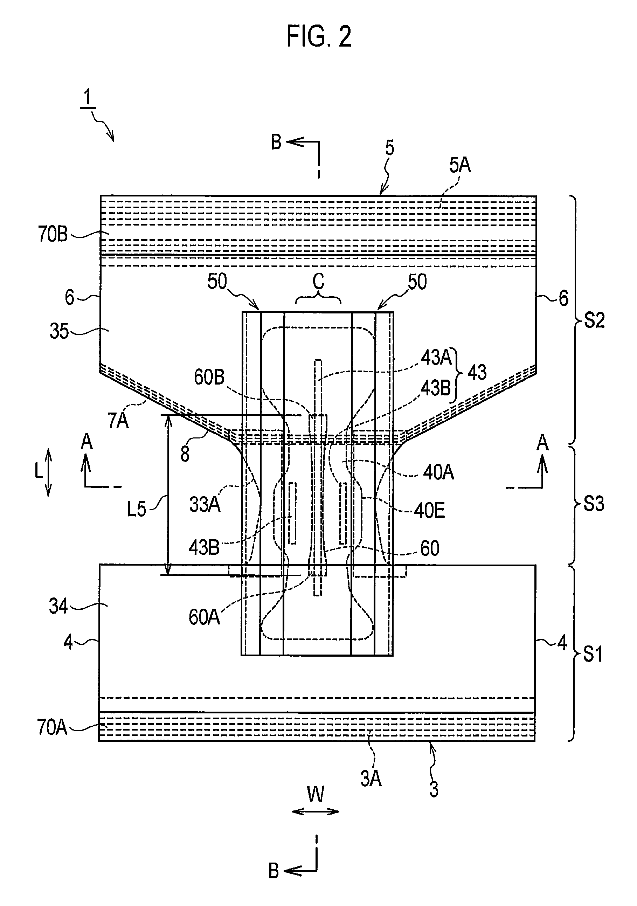

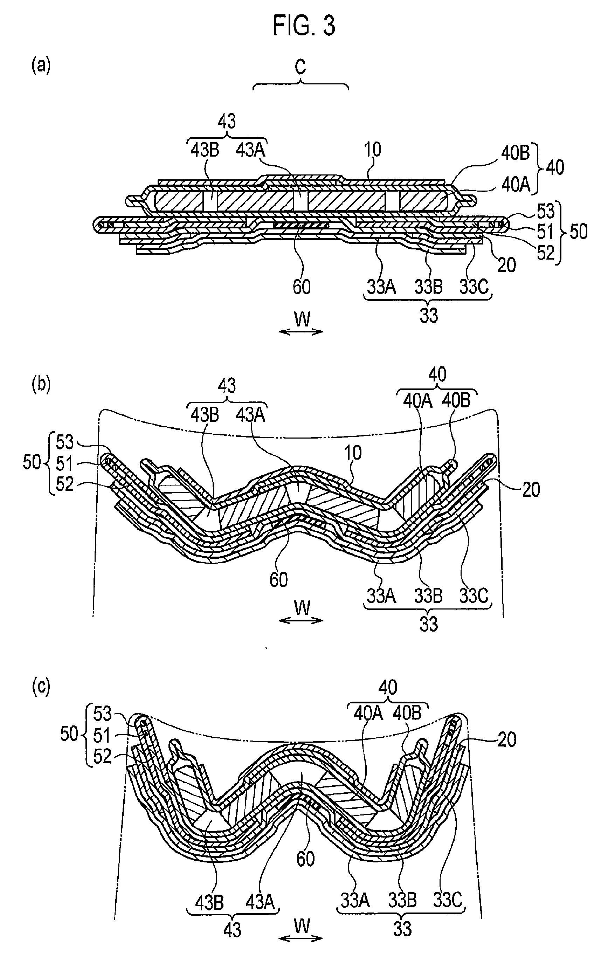

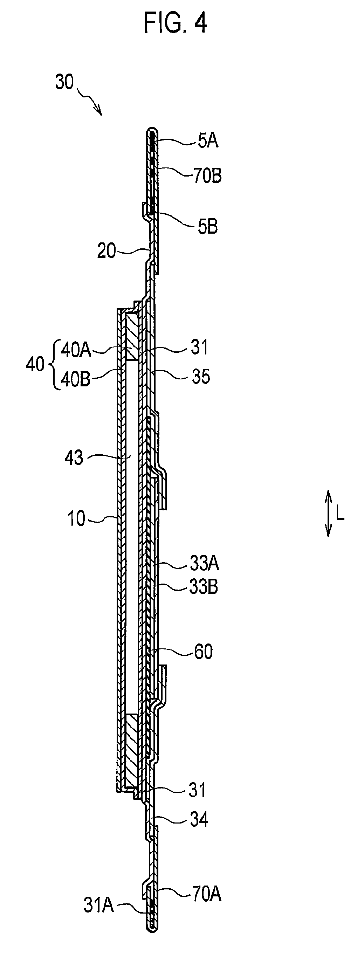

First, with reference to the drawings, a configuration of an absorbent article 1 according to a first embodiment will be described. Fig. 1 is an exploded perspective view showing the absorbent article 1 according to the first embodiment. Fig. 2 is a plan view showing the absorbent article 1 according to the first embodiment. Fig. 3 is a cross-sectional view (cross-sectional view taken along the line A-A in Fig. 2) showing the absorbent article 1 according to the first embodiment. Fig. 4 is a cross-sectional view (cross-sectional view taken along the line B-B in Fig. 2) showing the absorbent article 1 according to the first embodiment. Note that the absorbent article 1 is a shorts-type diaper in the first embodiment.

As shown in Figs. 1 to 4, the absorbent article 1 has a long shape in a front-back direction (hereinafter referred to as a longitudinal direction L of the absorbent article 1) which corresponds to a direction from a front side to a rear side of a wearer.

The absorbent article 1 has, in the longitudinal direction L of the absorbent article 1, a front waistline region S1 corresponding to a front waistline of the wearer, a rear waistline region S2 corresponding to a rear waistline of the wearer, and a crotch region S3 corresponding to a crotch of the wearer and positioned between the front waistline region S1 and the rear waistline region S2.

In the front waistline region S1, a waist gathering 3 is provided. In the waist gathering 3, multiple long and narrow waist elastic bodies 3A such as synthetic rubber are provided so as to stretch in a width direction W perpendicular to the longitudinal direction L of the absorbent article 1. The waist elastic bodies 3A are provided in a state of being stretched in the width direction W of the absorbent article 1. The waist elastic bodies 3A continuously extends from one front waistline edge 4 to the other front waistline edge 4, the front waistline edges 4 each positioned on a lateral side in the width direction W of the absorbent article 1 in the front waistline region S1.

In the rear waistline region S2, a waist gathering 5 is provided. In the waist gathering 5, multiple long and narrow waist elastic bodies 5A such as synthetic rubber are provided so as to stretch in the width direction W of the absorbent article 1. The waist elastic bodies 5A are provided in a state of being stretched in the width direction W of the absorbent article 1. The waist elastic bodies 5A continuously extends from one rear waistline edge 6 to the other rear waistline edge 6, the front waistline edges 4 each positioned on a lateral side in the width direction W of the absorbent article 1 in the rear waistline region S2.

In addition, a leg gathering 7 is also provided in the rear waistline region S2. In the leg gathering 7, multiple long and narrow leg elastic bodies 7A (first elastic bodies) such as synthetic rubber are provided along the width direction W of the absorbent article 1. The leg elastic bodies 7A are bent along a crotch edge 8 positioned on the crotch region S3 side in the rear waistline region S2, and continuously extend from one rear waistline edge 6 to the other rear waistline edge 6.

The absorbent article 1 as described above includes a topsheet 10, an outer topsheet 20, an outer sheet 30 (see Fig. 1), an absorber 40, a leakage preventive part 50, and a central elastic body 60 (second elastic body).

The topsheet 10 is provided closer to a side that comes into contact with the skin of the wearer than the absorber 40. The topsheet 10 is formed of a liquid-permeable sheet such as a hydrophilic nonwoven fabric or woven fabric, an apertured plastic film and an apertured hydrophobic nonwoven fabric.

The outer topsheet 20 is provided between the outer sheet 30 and the absorber 40. Moreover, the outer topsheet 20 is provided in the front waistline region S1, the rear waistline region S2 and the crotch region S3. The outer topsheet 20 is formed of a sheet such as a nonwoven fabric.

When in use, the outer sheet 30 is positioned on an outer side, i.e. located away from the skin of the wearer. The outer sheet 30 consists of: a front outer sheet 31 provided in the front waistline region S1; a rear outer sheet 32 positioned in the rear waistline region S2; a crotch outer sheet 33 positioned in the crotch region S3; a front waistline outer sheet 34 to which the front outer sheet 31 is attached; and a rear waistline outer sheet 35 to which the rear outer sheet 32 is attached.

The front outer sheet 31 is formed of a waterproof film (e.g., polyethylene) or the like. The rear outer sheet 32 is formed of a liquid-nonpermeable sheet such as a waterproof film.

The crotch outer sheet 33 includes: a liquid-nonpermeable crotch inner sheet 33A such as a waterproof film; a crotch outer layer sheet 33B, such as a nonwoven fabric, which is provided at a position farther from the wearer than the crotch inner sheet 33A; and a crotch sheet 33C, such as a nonwoven fabric, which is provided between the outer topsheet 20 and the crotch inner sheet 33A.

The front waistline outer sheet 34 is formed of a stretchable sheet. A front waist sheet 70A, such as a nonwoven fabric, which covers the waist elastic bodies 3A is connected to the front waistline outer sheet 34.

The rear waistline outer sheet 35 is formed of a stretchable sheet. A rear waist sheet 70B, such as nonwoven fabric, which covers the waist elastic bodies 5A is connected to the rear waistline outer sheet 35.

The absorber 40 is provided between the topsheet 10 and the outer sheet 30. The absorber 40 is formed of a powder mix 40A such as crushed pulp and high-absorbent polymer, and a covering material 40B, such as a hydrophilic nonwoven fabric, which covers the powder mix 40A. Note that the absorber 40 will be described in detail later.

The leakage preventive part 50 is provided along the longitudinal direction L of the absorbent article 1 on both sides 40E of the absorber 40. The leakage preventive part 50 includes leakage preventive elastic bodies 51, a leakage preventive film sheet 52 and a leakage preventive nonwoven fabric sheet 53.

The leakage preventive elastic body 51 is formed of synthetic rubber or the like that is stretchable along the longitudinal direction L of the absorbent article 1 on the both sides 40E of the absorber 40. Between the folded leakage preventive nonwoven fabric sheet 53, multiple leakage preventive elastic bodies 51 are provided along the longitudinal direction L of the absorbent article 1 in a state of being stretched in the longitudinal direction L of the absorbent article 1.

The leakage preventive film sheet 52 is formed of a liquid-nonpermeable sheet such as a waterproof film (e.g., polyethylene). The leakage preventive film sheet 52 is provided on an inner side of the leakage preventive elastic bodies 51 in the width direction W of the absorbent article 1. The leakage preventive nonwoven fabric sheet 53 is formed of a sheet such as a nonwoven fabric, which wraps around parts of the leakage preventive elastic bodies 51 and the leakage preventive film sheet 52.

The central elastic body 60 is a sheet such as a nonwoven fabric, which is provided along the longitudinal direction L of the absorbent article 1 and is stretchable along the longitudinal direction L of the absorbent article 1. The central elastic body 60 is provided at a position corresponding to a central portion C in the width direction W of the absorbent article 1 in a state of being stretched along the longitudinal direction L of the absorbent article 1. Moreover, the central elastic body 60 is provided between the outer topsheet 20 and the outer sheet 30. In the first embodiment, the central elastic body 60 is provided between the outer topsheet 20 and the crotch inner sheet 33A of the crotch outer sheet 33.

Note that the central elastic body 60 is not necessarily provided between the outer topsheet 20 and the outer sheet 30, but may be provided anywhere between the crotch outer sheet 33 and the absorber 40. For example, the central elastic body 60 may be provided between an inner layer sheet (such as the crotch inner sheet 33A) and an outer layer sheet (the crotch outer layer sheet 33B), which make up the crotch outer sheet 33. The central elastic body 60 will be described in detail later.

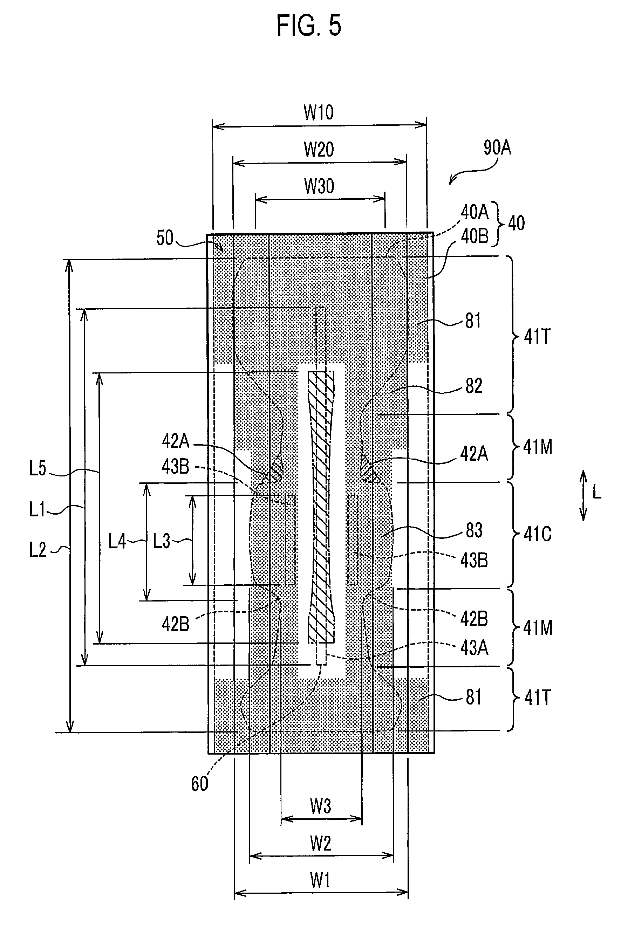

Next, with reference to the drawings, a configuration of the above-described absorber 40 will be described. Fig. 5 is a plan view showing a first crotch member 90A according to the first embodiment. Note that the first crotch member 90A includes the absorber 40 (the powder mix 40A and the covering material 40B) and the leakage preventive part 50 (the leakage preventive elastic bodies 51, the leakage preventive film sheet 52 and the leakage preventive nonwoven fabric sheet 53).

As shown in Fig. 5, in a planar view of the absorbent article 1 (the absorber 40), the powder mix 40A has a pair of waistline portions 41T, a central crotch portion 41C and a pair of intermediate crotch portions 41M.

The waistline portions 41T are positioned in a pair of waistline regions (the front waistline region S1 and the rear waistline region S2). A width (W1) of the waistline portions 41T along the width direction W of the absorbent article 1 is larger than a width (W2) of the central crotch portion 41C or a width (W3) of the intermediate crotch portions 41M.

The central crotch portion 41C is positioned in the center of the crotch region S3 in the longitudinal direction L of the absorbent article 1. The width (W2) of the central crotch portion 41C along the width direction W of the absorbent article 1 is smaller than the width (W1) of the waistline portions 41T and larger than the width (W3) of the intermediate crotch portions 41M.

The intermediate crotch portions 41M are each positioned between the waistline portion 41T and the central crotch portion 41C. The width (W3) of the intermediate crotch portions 41M along the width direction W of the absorbent article 1 is smaller than the width (W1) of the waistline portions 41T or the width (W2) of the central crotch portion 41C.

Here, in the planar view of the absorbent article 1, connection parts 42A between the intermediate crotch portion 41M positioned on the rear waistline region S2 side and the central crotch portion 41C are connected more smoothly than connection parts 42B between the intermediate crotch portion 41M positioned on the front waistline region S1 side and the central crotch portion 41C.

Such a powder mix 40A has a bent part 43 formed along the longitudinal direction L of the absorbent article 1. The bent part 43 has a slit shape penetrating the powder mix 40A, and includes a central bent portion 43A and a pair of side bent portions 43B.

The central bent portion 43A is formed in a position corresponding to the central portion C in the width direction W of the absorbent article 1. A length (L1) of the central bent portion 43A along the longitudinal direction L of the absorbent article 1 is smaller than a length (L2) of the powder mix 40A along the longitudinal direction L of the absorbent article 1. The central bent portion 43A is formed to spread from one of the intermediate crotch portions 41M to the other of the intermediate crotch portions 41M via the central crotch portion 41C.

Each of the side bent portions 43B is formed on an outer side of the central bent portion 43A in the width direction W of the absorbent article 1. Each of the side bent portions 43B is provided between a corresponding one of the both sides 40E of the absorber 40 and the central elastic body 60 (the central bent portion 43A), while being provided within the central crotch portion 41C. A length (L3) of the side bent portions 43B along the longitudinal direction L of the absorbent article 1 is smaller than a length (L4) of the central crotch portion 41C along the longitudinal direction L of the absorbent article 1.

Next, with reference to Figs. 1 to 5, a configuration of the above-described central elastic body 60 will be described. As shown in Figs. 1 to 5, the central elastic body 60 overlaps with the front waistline region S1 and the rear waistline region S2 in a thickness direction T of the absorbent article 1. To be more specific, the central elastic body 60 has a front end portion 60A positioned on the front waistline region S1 side and a rear end portion 60B positioned on the rear waistline region S2 side. In other words, the front end portion 60A overlaps with the front waistline region S1 in the thickness direction T of the absorbent article 1. Meanwhile, the rear end portion 60B overlaps with the rear waistline region S2 in the thickness direction T of the absorbent article 1.

Moreover, at least a part of the central elastic body 60 intersects with the leg elastic bodies 7A. The central elastic body 60 overlaps with the central bent portion 43A in the thickness direction T of the absorbent article 1. A length (L5) of the central elastic body 60 along the longitudinal direction L of the absorbent article 1 is smaller than the length (L1) of the central bent portion 43A along the longitudinal direction L of the absorbent article 1 (see Fig. 5).

Next, with reference to Fig. 5, a description will be given of a configuration of a joint 80 which joins the members that make up the absorbent article 1 described above to the first crotch member 90A. Note that the joint 80 indicates an area where the members that make up the absorbent article 1 and the first crotch member 90A are joined with an adhesive (e.g., hot-melt) or the like.

The joint 80 includes a top-side joint (not shown) and an outer-side joint (not shown). The top-side joint indicates a portion where a top-side sheet (the topsheet 10 in this embodiment), which is positioned closer to the topsheet 10 side than the first crotch member 90A, and the first crotch member 90A are joined. On the other hand, the outer-side joint indicates a portion where an outer-side sheet (the outer topsheet 20 in this embodiment), which is positioned closer to the outer sheet 30 side than the first crotch member 90A, and the first crotch member 90A are joined.

A description will be given below by taking the outer-side joint (hereinafter simply referred to as the joint 80) as an example. In the planar view of the absorbent article 1, the joint 80 consists of a wide joint 81, an intermediate joint 82 continuous with the wide joint 81, and a narrow joint 83 continuous with the intermediate joint part 82.

The wide joint 81 is provided in the pair of waistline regions (the front waistline region S1 and the rear waistline region S2). Specifically, the wide joint 81 is provided in the pair of waistline portions 41T of the absorber 40. A width (W10) of the wide joint 81 along the width direction W of the absorbent article 1 is larger than a width (W20) of the intermediate joint 82 along the width direction W of the absorbent article 1 or a width (W30) of the narrow joint 83 along the width direction W of the absorbent article 1.

The intermediate joint 82 is provided at a position corresponding to the leg elastic bodies 7A in the rear waistline region S2. Specifically, the intermediate joint 82 is provided in the intermediate crotch portion 41M of the absorber 40, which is positioned on the rear waistline region S2 side. The width (W20) of the intermediate joint 82 is smaller than the width (W10) of the wide joint 81 and larger than the width (W30) of the narrow joint 83.

The narrow joint 83 is provided in the crotch region S3. Specifically, the narrow joint 83 is provided in the intermediate crotch portion 41M of the absorber 40, which is positioned on the front waistline region S1 side, and in the central crotch portion 41C of the absorber 40. The width (W30) of the narrow joint 83 is smaller than the width (W10) of the wide joint 81 or the width (W20) of the intermediate joint 82.

Here, in the intermediate joint 82 and the narrow joint 83, at least a portion where the central bent portion 43A is provided is not joined. For this reason, the central elastic body 60 described above is provided so as to be shifted from the joint 80 (the top-side joint and the outer-side joint) in the width direction W of the absorbent article 1. In other words, the central elastic body 60 does not overlap with the joint 80 in the thickness direction T of the absorbent article 1.

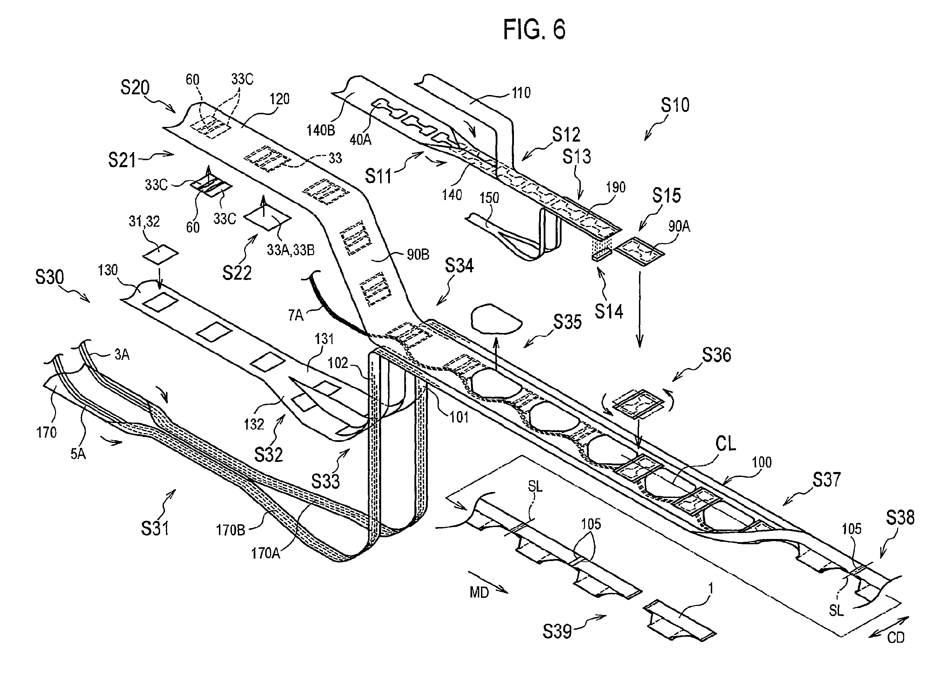

Next, with reference to the drawings, a description will be given of a method for manufacturing an absorbent article according to the first embodiment. Fig. 6 is a view for illustrating a method for manufacturing an absorbent article according to the first embodiment. Note that, in Fig. 6, steps of applying an adhesive other than a later-described adhesive application step S14 are omitted.

As shown in Fig. 6, the method for manufacturing an absorbent article includes: a first crotch member manufacturing process S10 for manufacturing members such as the absorber 40 and the leakage preventive part 50; a second crotch member manufacturing process S20 for manufacturing members such as the central elastic body 60 and the crotch outer sheet 33; and a main body manufacturing process S30 for manufacturing a front waistline member which constitutes the front waistline region S1, a rear waistline member that constitutes the rear waistline region S2, and the like.

First, the first crotch member manufacturing process S10 will be described with reference to Fig. 6. As shown in Fig. 6, the first crotch member manufacturing process S10 includes an absorber formation step S11, a topsheet attachment step S12, a leakage preventive part attachment step S13, the adhesive application step S14, and a crotch member cutting step S15.

In the absorber formation step S11, a covering material sheet 140B of the continuum of the covering materials 40B is wrapped around the powder mixes 40A, each having the bent part 43 (see Figs. 1 to 5) formed therein, thereby forming an absorber web 140 of the continuum of the absorbers 40. Note that the bent part 43 is formed along a conveying direction MD of the absorber web 140.

In the topsheet attachment step S12, a topsheet web 110 of the continuum of the topsheets 10, each constituting the crotch region S3, is attached onto the absorber web 140.

In the leakage preventive part attachment step S13, a leakage preventive part web 150 of the continuum of the leakage preventive parts 50, each being previously formed of the leakage preventive elastic bodies 51, the leakage preventive film sheet 52 and the leakage preventive nonwoven fabric sheet 53, is attached onto portions on a lower surface side of the absorber web 140 corresponding to both of the sides 40E of the absorber 40, along the conveying direction MD of the absorber web 140. Thus, a first crotch member web 190 of the continuum of the first crotch members 90A is formed.

In the adhesive application step S14, an adhesive is applied to the first crotch member web 190. Note that the adhesive is applied to portions corresponding to the above-described wide joint 81, intermediate joint 82 and narrow joint 83 (see Fig. 5).

In the crotch member cutting step S15, the first crotch member web 190 having applied the adhesive is cut at predetermined intervals (i.e. into a size of the first crotch member 90A as a product). Thus, in the first crotch member manufacturing process S10, the first crotch member 90A including the absorber 40, the leakage preventive part 50 and the like is manufactured.

Next, the second crotch member manufacturing process S20 will be described with reference to Fig. 6. As shown in Fig. 6, the second crotch member manufacturing process S20 includes a central elastic body attachment step S21 and a crotch outer sheet attachment step S22.

In the central elastic body attachment step S21, the central elastic body 60 is attached, in a state of being stretched, to a lower surface side of an outer topsheet web 120 of the continuum of the outer topsheets 20 along an orthogonal direction CD perpendicular to the conveying direction MD of the outer topsheet web 120. In this event, the crotch sheet 33C is attached together with the central elastic body 60.

In the crotch outer sheet attachment step S22, the crotch inner sheet 33A and the crotch outer layer sheet 33B are attached to the outer topsheet web 120 so that the central elastic body 60 is sandwiched between the outer topsheet web 120 and the sheets 33A and 33B. Thus, in the second crotch member manufacturing process S20, a second crotch member 90B including the central elastic body 60, the crotch outer sheet 33 and the like is manufactured.

Note that the crotch outer sheet attachment step S22 may be performed simultaneously with the central elastic body attachment step S21.

Next, the main body manufacturing process S30 will be described with reference to Fig. 6. As shown in Fig. 6, the main body manufacturing process S30 includes a waist sheet formation step S31, a waistline outer sheet formation step S32, a waistline member formation step S33, a second crotch member attachment step S34, a leg girth cutting step S35, a first crotch member attachment step S36, a folding step S37, an edge joining step S38, and an end cutting step S39.

In the waist sheet formation step S31, on a waist web 170 of the continuum of the waist sheets (the front waist sheet 70A and the rear waist sheet 70B), the waist elastic bodies 3A and the waist elastic bodies 5A are attached, in a state of being stretched, along the conveying direction MD of the waist web 170.

Next, both edges of the waist web 170 are folded to wrap the waist elastic bodies 3A and the waist elastic bodies 5A therein, respectively. Thereafter, the waist web 170 is cut along the conveying direction MD of the waist web 170 so as to correspond to the front waistline region S1 and the rear waistline region S2. Thus, a front waist web 170A (front waistline web) of the continuum of the front waist sheets 70A and a rear waist web 170B (rear waistline web) of the continuum of the rear waist sheets 70B are formed.

In the waistline outer sheet formation step S32, a sheet having the front outer sheet 31 and rear outer sheet 32 integrated therein is attached to a waistline outer sheet web 130 of the continuum of the waistline sheets. Thereafter, the waistline outer sheet web 130 is cut along a conveying direction MD of the waistline outer sheet web 130 so as to correspond to the front waistline region S1 and the rear waistline region S2. Thus, a front outer sheet web 131 having the front outer sheets 31 attached thereto and a rear outer sheet web 132 having the rear outer sheets 32 attached thereto are formed.

In the waistline member formation step S33, the front waist web 170A formed in the waist sheet formation step S31 is attached to a lower surface side of the front outer sheet web 131 formed in the waistline outer sheet formation step S32. Meanwhile, the rear waist web 170B formed in the waist sheet formation step S31 is attached to a lower surface side of the rear outer sheet web 132 formed in the waistline outer sheet formation step S32. Thus, a front waistline web 101 of the continuum of the front waistline members, each constituting the front waistline region S1, and a rear waistline web 102 of the continuum of the rear waistline members, each constituting the rear waistline region S2, are formed.

In the second crotch member attachment step S34, the second crotch member 90B including the central elastic body 60, the crotch outer sheet 33 and the like formed in the second crotch member manufacturing process S20 described above is attached between the front waistline web 101 and the rear waistline web 102. At the same time, between the rear waistline web 102 and the second crotch member 90B, the leg elastic bodies 7A are bonded in a state where at least a part of the leg elastic bodies 7A are stretched while swinging the leg elastic bodies 7A in the orthogonal direction CD.

In this event, between the front waistline web 101 and the rear waistline web 102, the central elastic body 60 is disposed so as to correspond to the central portion C in the width direction W of the absorbent article 1. Moreover, the central elastic body 60 is caused to overlap with at least one of the front waistline web 101 and the rear waistline web 102 in the thickness direction T of the absorbent article 1. Furthermore, at least a part of the central elastic body 60 is caused to intersect with the leg elastic bodies 7A (not shown).

Here, in the waistline member formation step S33 and the second crotch member attachment step S34, the front waist web 170A and the rear waist web 170B may be attached after attaching the front outer sheet web 131 and the rear outer sheet web 132 to the second crotch member 90B.

In the leg girth cutting step S35, positions corresponding to the leg girth of the wearer, i.e. the front waistline web 101, the rear waistline web 102, the outer topsheet web 120 and the crotch outer sheet 33 are cut at predetermined intervals in the conveying direction MD.

In the first crotch member attachment step S36, the first crotch member 90A including the absorber 40, the leakage preventive part 50 and the like formed in the first crotch member manufacturing process S10 described above is attached to the opposite side of the outer topsheet web 120 having the front waistline web 101 and rear waistline web 102 attached thereto. In this event, the first crotch member 90A is attached so that the central bent portion 43A overlaps with at least a part of the central elastic body 61. As a result, the absorber 40 having the bent part 43 formed therein is disposed between the front waistline web 101 and the rear waistline web 102. Thus, a product web 100 of the continuum of the absorbent articles 1 is formed.

In the folding step S37, the product web 100 is folded at a folding position (straight line CL) along the conveying direction MD of the product web 100 in such a manner that the rear waistline web 102 is brought toward the front waistline web 101. Note that the folding position may be, for example, the center of the product web 100 in the orthogonal direction CD or may be shifted from the straight line CL toward the front waistline web 101 or toward the rear waistline web 102.

In the edge joining step S38, joints 105 corresponding to the front waistline edges 4 and the rear waistline edges 6 are joined at predetermined intervals in the conveying direction MD of the product web 100 by ultrasonic treatment or heat treatment. Note that the joints 105 indicate both sides of a virtual line SL which is extended in the orthogonal direction CD of the product web 100 and which indicates a position at which the product web 100 is to be cut.

In the end cutting step S39, the product web 100 having the joints 105 formed therein is cut along the position at which the product web 100 is to be cut (the virtual line SL) . Thus, the absorbent article 1 is manufactured.

In the first embodiment described above, at least a part (the rear end portion 60B) of the central elastic body 60 intersects with the leg elastic bodies 7A, and overlaps with the central bent portion 43A in the thickness direction T of the absorbent article 1. Thus, when the absorbent article is worn, even if the front end portion 60A and rear end portion 60B of the central elastic body 60 are to contract toward the center of the crotch region S3 in a direction of reducing a distance between the end portions 60A and 60B, the central elastic body 60 is held by the leg elastic bodies 7A continuous with the crotch edge 8. For this reason, even in a state where bodily waste of the wearer is received, the stomach-side portion and back-side portion of the absorbent article 1 are less likely to come down toward the crotch of the wearer. Therefore, the absorbent article 1 can be prevented from separating from the skin of the wearer, and thus is more comfortably worn by the wearer. Moreover, since the absorber 40 can be prevented from moving away from the skin of the wearer, the bodily waste can be absorbed by the absorber 40 immediately after being excreted.

The bent part 43 (the central bent portion 43A and the side bent portions 43B) is particularly provided in the absorber 40. With this configuration, the central portion C of the absorber 40 protrudes toward the wearer with the central bent portion 43A as a base point and the outer sides (outer portions) of the central portion C sags in a direction of separating from the wearer (downward) (see Figs. 3 (b) and 3 (c)). Thus, the central portion C is attached to the wearer, and the bodily waste of the wearer can be quickly absorbed. Moreover, the absorbent article 1 more readily fits the crotch of the wearer, and thus is more comfortably worn by the wearer.

Moreover, the central elastic body 60 overlaps with the central bent portion 43A in the thickness direction T of the absorbent article 1. Therefore, compared with the case where the central elastic body 60 does not overlap with the central bent portion 43A, the absorbent article 1 is more readily bent. Thus, the tension of the central elastic body 60 causes the absorber 40 to be more readily bent with the central bent portion 43A as the base point, and facilitates deformation of the central portion C in the width direction W of the absorbent article 1. As a result, the absorbent article 1 even more readily fits the wearer (particularly at the crotch).

In the first embodiment, the length (L5) of the central elastic body 60 is smaller than the length (L1) of the central bent portion 43A. Therefore, the central elastic body 60 fits within the central bent portion 43A, and the central elastic body 60 does not overlap with the powder mix 40A. This can prevent the powder mix 40A from getting wrinkled. Note that, if the length (L5) of the central elastic body 60 is larger than the length (L1) of the central bent portion 43A, the central elastic body 60 overlaps with the powder mix 40A. As a result, the absorbent article 1 gets wrinkled and is increased in thickness. For this reason, the absorbent article 1 is less likely to be deformed and may be less comfortably worn by the wearer. For example, if the central elastic body 60 is long on the front waistline region S1 side, the stomach of the wearer is compressed (more likely to be compressed in male users) and the powder mix 40A is likely to get wrinkled. Hence, the absorbent article 1 may be less comfortably worn by the wearer.

In the first embodiment, the leakage preventive part 50 (the leakage preventive elastic bodies 51) is provided in the absorbent article 1. When the absorbent article 1 is worn by the wearer, the leakage preventive part 50 stands up toward the crotch of the wearer on both of the sides 40E of the absorber 40 to form barriers. Thus, the absorbent article 1 fits the wearer more readily than the case where the leakage preventive part 50 is not provided. Therefore, the absorbent article 1 is more comfortably worn by the wearer while leakage is prevented at the sides.

In the first embodiment, the bent part 43 has the central bent portion 43A and the pair of side bent portions 43B. This causes the central portion C of the absorber 40 to readily protrude toward the wearer with the central bent portion 43A as the base point. Meanwhile, the outer sides (outer portions) of the central portion C sags in the direction of separating from the wearer, and the leakage preventive part 50 stands up toward the wearer with the side bent portions 43B as base points. Thus, barriers are formed on both of the sides 40E of the absorber 40, and a so-called W-shape (see Figs. 3 (b) and 3 (c)) is formed. Therefore, even when the bodily waste of the wearer flows outward in the width direction W of the absorbent article 1, the bodily waste is blocked on both of the sides 40E of the absorber 40.

In the first embodiment, each of the side bent portions 43B is provided on a position inward than a corresponding one of both of the sides 40E of the absorber 40 in the width direction W of the absorbent article 1. This causes the ends of the absorber 40 to readily stand up with the side bent portions 43B as the base points. Thus, the absorber 40 readily fits the crotch of the wearer.

In the first embodiment, the central elastic body 60 is provided so as to be shifted from the joint 80 (the top-side joint and the outer-side joint) in the thickness direction T of the absorbent article 1. Note that, when the central elastic body 60 overlaps with the joint 80, contraction of the central elastic body 60 may cause the powder mix 40A to get wrinkled.

In the first embodiment, the width (W1) of the waistline portions 41T is larger than the width (W2) of the central crotch portion 41C or the width (W3) of the intermediate crotch portions 41M, while the width (W2) of the central crotch portion 41C is larger than the width (W3) of the intermediate crotch portions 41M. Thus, the area for absorbing the bodily waste of the wearer can be increased. Moreover, the width (W3) of the intermediate crotch portions 41M is smaller than the width (W1) of the waistline portions 41T or the width (W2) of the central crotch portion 41C. Thus, capability of following the movement of the wearer is enhanced. Specifically, since the powder mix 40A has the waistline portions 41T, the central crotch portion 41C and the intermediate crotch portions 41M, both absorbability for the bodily waste of the wearer and the capability of following the movement of the wearer can be achieved.

Particularly, the connection parts 42A between the intermediate crotch portion 41M positioned on the rear waistline region S2 side and the central crotch portion 41C are connected more smoothly than connection parts 42B between the intermediate crotch portion 41M positioned on the front waistline region S1 side and the central crotch portion 41C. Thus, the area for absorbing the bodily waste of the wearer can be increased. The absorbability for the bodily waste particularly when the wearer lies on his/her back is enhanced.

In the first embodiment, the joint 80 (outer-side joint) consists of the wide joint 81, the intermediate joint 82 and the narrow joint 83. Since the wide joint 81 is provided in the waistline portions 41T, the first crotch member 90A can be prevented from being flipped or folded. Thus, the absorbent article 1 is more comfortably worn by the wearer. Incidentally, in order to enhance the absorbability for the bodily waste of the wearer, the width (W30) of the narrow joint 83 is set smaller than the width (W20) of the intermediate joint 82. In other words, by setting the width (W30) of the narrow joint 83 small, the leakage preventive part 50 provided on the both sides 40E of the absorber 40 can be more readily stood up. Moreover, by setting the width (W20) of the intermediate joint 82 larger than the width (W30) of the narrow joint 83, an excessive increase in height of the leakage preventive part 50 can be suppressed. Furthermore, at the buttocks-side or the like of the crotch, the tip of the leakage preventive part 50 can be prevented from falling inward in the width direction W of the absorbent article 1. Therefore, the leakage preventive part 50 can be prevented from covering the absorber 40. Thus, both stable absorption performance and comfort in wearing can be achieved.

In the first embodiment, the central elastic body 60 is provided between the outer topsheet 20 and the outer sheet 30. In other words, the central elastic body 60 is provided closer to the outer sheet 30 side than the absorber 40. Thus, the absorber 40 itself is lifted toward the wearer by the tension of the central elastic body 60, and the absorber 40 more readily fits the wearer (particularly at the crotch). Moreover, compared with the case where the central elastic body 60 is provided between the topsheet 10 and the absorber 40, for example, the topsheet 10 is less likely to get wrinkled.

In the first embodiment, the central elastic body 60 is a sheet such as a nonwoven fabric which is stretchable along the longitudinal direction L of the absorbent article 1. Thus, compared with the case where the central elastic body 60 is formed of long and narrow synthetic rubber, the absorber 40 and the like can be pressed down in a planar manner. Thus, the central elastic body 60 can be surely located overlapping with the central bent portion 43A. In other words, a larger area of the central elastic body 60 overlapping with the central bent portion 43A can be secured. Therefore, since the absorber 40 can protrude more stably toward the wearer, the absorbent article 1 can be more surely attached to the skin of the wearer.

### (Modified Examples)

The absorbent article 1 according to the above-described first embodiment may be modified as described below. Note that the same parts as those of the absorbent article 1 according to the above-described first embodiment are denoted by the same reference numerals, and different parts will be mainly described.

### (Modified Example 1)

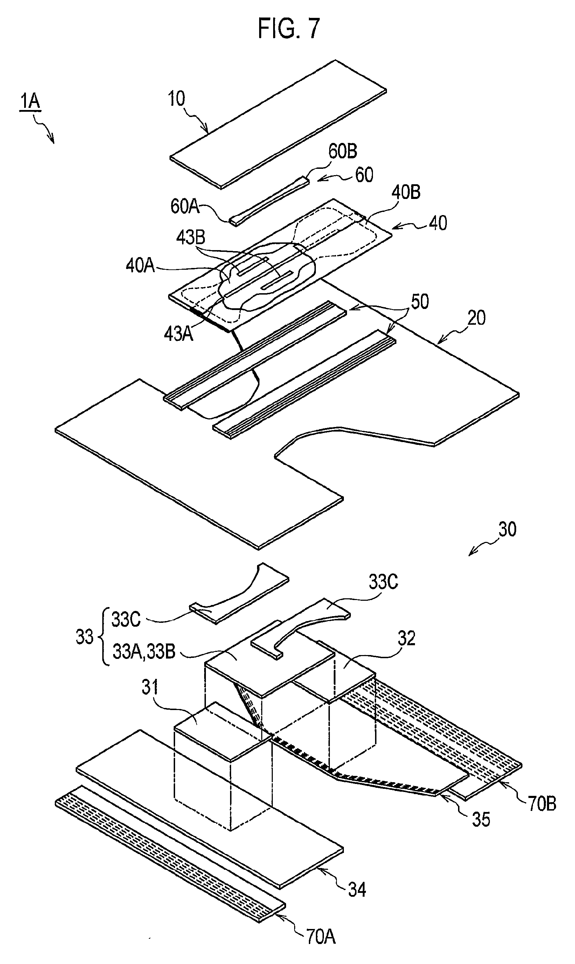

First, with reference to the drawings, an absorbent article 1A according to modified example 1 will be described. Fig. 7 is an exploded perspective view showing the absorbent article 1A according to modified example 1.

In the absorbent article 1 according to the first embodiment described above, the central elastic body 60 is provided between the outer topsheet 20 and the outer sheet 30. Meanwhile, in the absorbent article 1A according to modified example 1 as shown in Fig. 7, a central elastic body 60 is provided between a topsheet 10 and an absorber 40. To be more specific, the central elastic body 60 is provided between the topsheet 10 and a covering material 40B of the absorber 40.

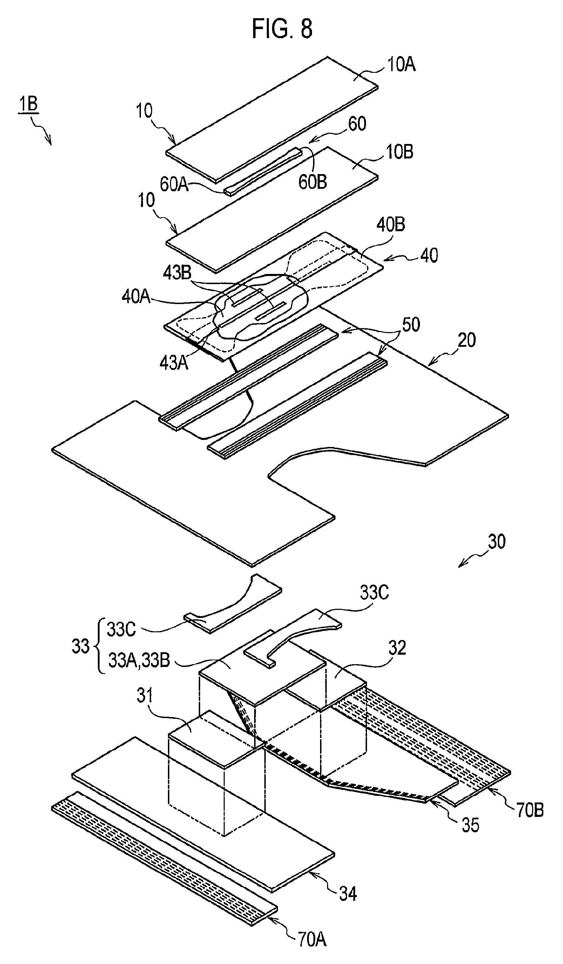

Here, as shown in Fig. 8, in an absorbent article 1B, a topsheet 10 may include: a top sheet 10A which comes into contact with the skin of the wearer; and a second sheet 10B provided closer to the absorber 40 side than the top sheet 10A. In this case, a central elastic body 60 may be provided between the top sheet 10A and the second sheet 10B.

Note that the central elastic body 60 is not necessarily provided between the top sheet 10A and the second sheet 10B, but may be provided anywhere between the top sheet 10A and the absorber 40. For example, the central elastic body 60 may be provided between the second sheet 10B and the covering material 40B.

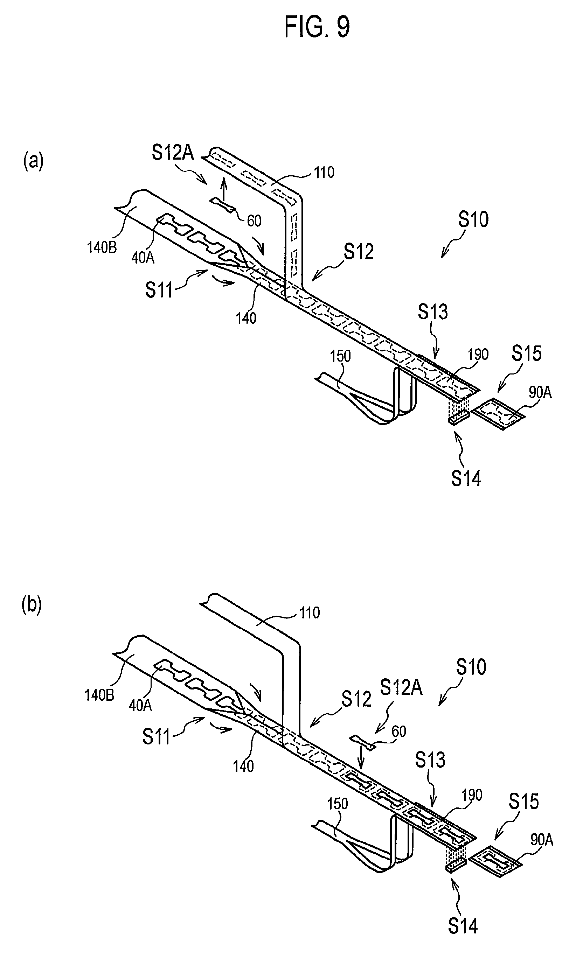

Next, with reference to the drawing, a description will be given of a method for manufacturing an absorbent article according to modified example 1. Fig. 9 is a view for illustrating the method for manufacturing an absorbent article according to modified example 1. Note that only steps different from those of the method for manufacturing the absorbent article according to the first embodiment will be described.

Here, in the method for manufacturing the absorbent article according to the first embodiment described above, after attaching the central elastic body 60 to the outer topsheet web 120, the absorber 40 is disposed so that the central bent portion 43A overlaps with the central elastic body 60. Meanwhile, in the method for manufacturing an absorbent article according to modified example 1, after disposing the central elastic body 60 between the topsheet web 110 and the absorber 40 (absorber web 140), at least the topsheet web 110 is attached to the absorber 40 so that the central elastic body 60 overlaps with the central bent portion 43A.

To be more specific, as shown in Fig. 9 (a), the topsheet attachment step S12 includes an elastic body disposition step S12A.

In the elastic body disposition step S12A, the central elastic body 60, which is stretchable along a conveying direction MD of the topsheet web 110, is disposed on the topsheet web 110 in a stretched state. Next, the topsheet web 110 is attached to the absorber web 140, so that the central elastic body 60 overlaps with at least a part of the central bent portion 43A.

Note that, in the first crotch member attachment step S36 of the main body manufacturing process S30, when attaching the first crotch member 90A to the outer topsheet web 120, the central elastic body 60 is caused to overlap with the front waistline web 101 and the rear waistline web 102 in the thickness direction T of the absorbent article 1. Moreover, at least a part of the central bent portion 43A is caused to intersect with the leg elastic bodies 7A.

Here, although the description has been given for the case where the central elastic body 60 is disposed on the topsheet web 110 in the above-described elastic body disposition step S12A, the present invention is not limited thereto. The central elastic body 60 may otherwise be disposed on the absorber web 140.

Moreover, although the description has been given for the case where the topsheet web 110 is attached to the absorber web 140 after the central elastic body 60 is disposed on the topsheet web 110 in the elastic body disposition step S12A, the present invention is not limited thereto. As shown in Fig. 9 (b), the central elastic body 60 may be disposed on the topsheet web 110 after the topsheet web 110 is attached to the absorber web 140. Moreover, when the topsheet 10 includes the top sheet 10A and the second sheet 10B, the central elastic body 60 may be disposed between a web of the continuum of the top sheets 10A and a web of the continuum of the second sheets 10B.

In modified example 1 described above, the central elastic body 60 is provided between the topsheet 10 and the absorber 40. Thus, for example, compared with the case where the central elastic body 60 is provided between the outer topsheet 20 and the outer sheet 30, the central elastic body 60 is disposed closer to the wearer. Therefore, the tension of the central elastic body 60 can be more exerted, which causes the top sheet 10A to more readily fit the wearer (particularly at the crotch).

### (Modified Example 2)

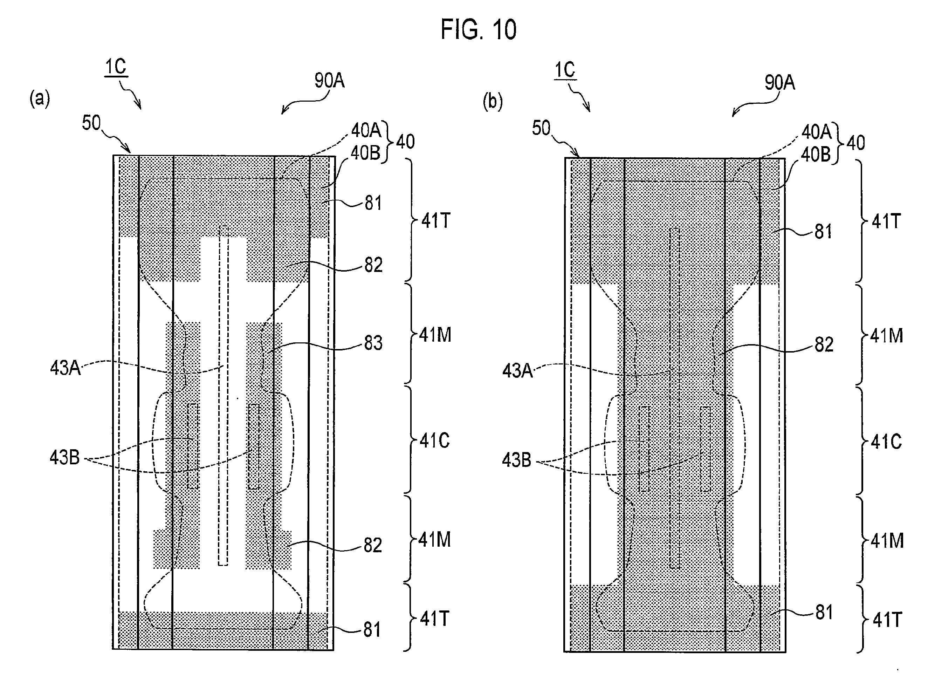

Next, with reference to the drawing, an absorbent article 1C according to modified example 2 will be described. Fig. 10 is a plan view showing a first crotch member 90A of the absorbent article 1C according to modified example 2.

In the absorbent article 1 according to the first embodiment described above, the wide joint 81, the intermediate joint 82 and the narrow joint 83 are connected to each other. Meanwhile, in the absorbent article 1C according to modified example 2, as shown in Fig. 10 (a), a wide joint 81, an intermediate joint 82 and a narrow joint 83 in a joint 80 are divided into segments.

Note that the joint 80 does not necessarily consist of the wide joint 81, the intermediate joint 82 and the narrow joint 83, but may consist of, for example, the wide joint 81 and the narrow joint 83 as shown in Fig. 10 (b). Moreover, in the joint 80, the portion where the bent part 43 is provided does not always have to be unconnected but may be connected.

### [Second Embodiment]

With reference to the drawings, a configuration of an absorbent article 1D according to a second embodiment will be described below. Note that the same parts as those of the absorbent article 1 according to the above-described first embodiment are denoted by the same reference numerals, and different parts will be mainly described.

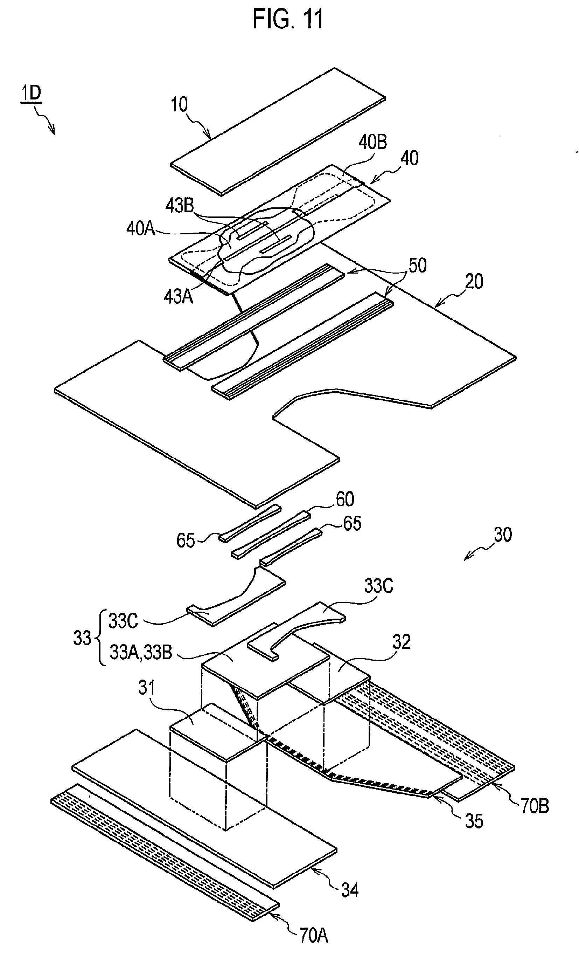

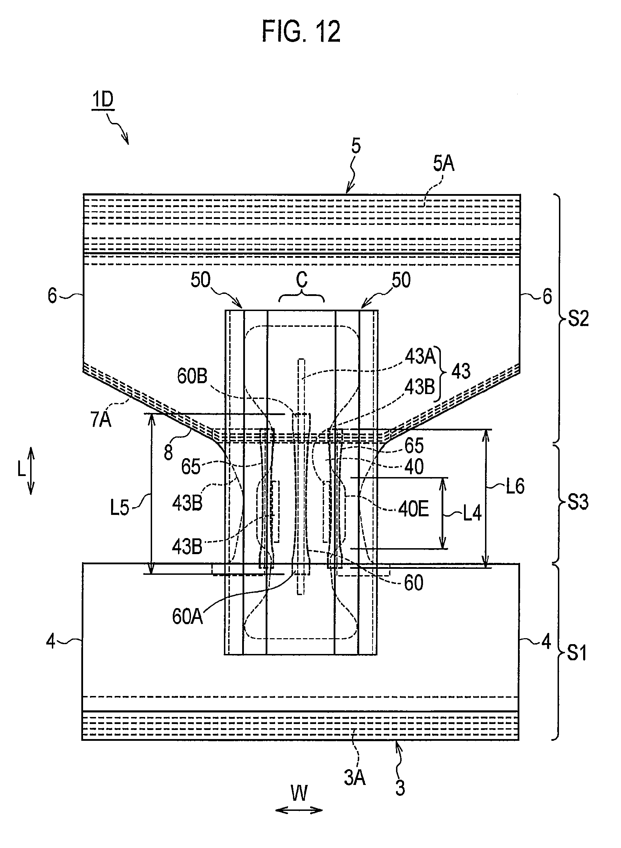

Fig. 11 is an exploded perspective view showing the absorbent article 1D according to the second embodiment. Fig. 12 is a plan view showing the absorbent article 1D according to the second embodiment. In the absorbent article 1 according to the first embodiment described above, one central elastic body 60 is provided. Meanwhile, in the absorbent article 1D according to the second embodiment, intermediate elastic bodies 65 (third elastic bodies) are provided in addition to a central elastic body 60. Note that, as in the case of the central elastic body 60, the intermediate elastic bodies 65 are sheets such as a nonwoven fabric which are stretchable along the longitudinal direction L of the absorbent article 1.

To be more specific, as shown in Figs. 11 and 12, the intermediate elastic bodies 65 are provided in a stretched state between the central elastic body 60 and leakage preventive elastic bodies 51. Moreover, as in the case of the central elastic body 60, the intermediate elastic bodies 65 are provided between an outer topsheet 20 and an outer sheet 30, more specifically, between the outer topsheet 20 and a crotch inner sheet 33A of a crotch outer sheet 33.

Each of the intermediate elastic bodies 65 is provided in a position outward than a corresponding one of the side bent portions 43B in the width direction W of the absorbent article 1D. In other words, each of the side bent portions 43B is provided in a position inward than a corresponding one of the intermediate elastic bodies 65 in the width direction W of the absorbent article 1D. Moreover, the intermediate elastic bodies 65 overlap with a central crotch portion 41C in the thickness direction T of the absorbent article 1D.

A length (L6) of the intermediate elastic bodies 65 along the longitudinal direction L of the absorbent article 1D is larger than a length (L4) of the central crotch portion 41C along the longitudinal direction L of the absorbent article 1D.

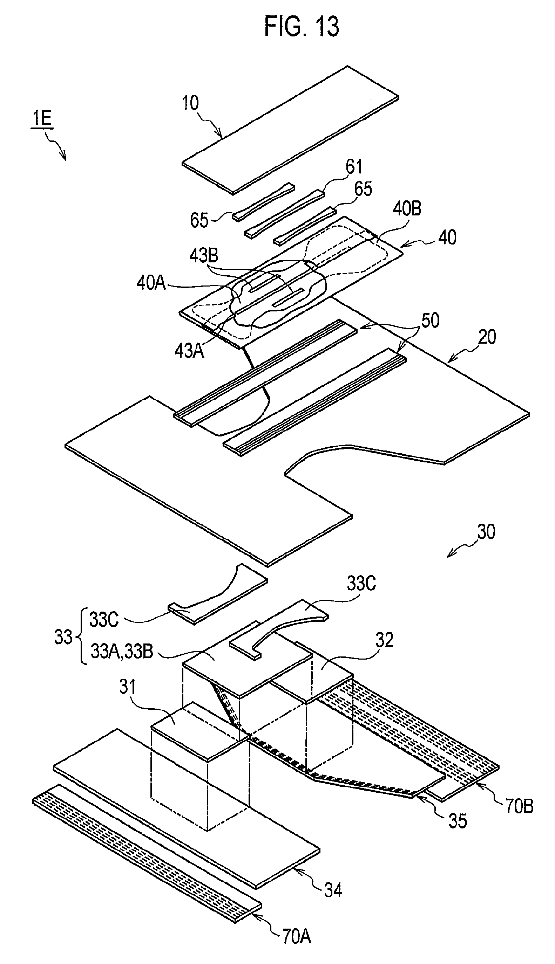

Here, the intermediate elastic bodies 65 are not necessarily provided between the outer topsheet 20 and the outer sheet 30. For example, as shown in Fig. 13, in an absorbent article 1E, intermediate elastic bodies 65 may be provided between a topsheet 10 and an absorber 40 as described in modified example 1 of the first embodiment. Moreover, the intermediate elastic bodies 65 are not necessarily provided in the same location as the central elastic body 60, but may be provided in a location away from the central elastic body 60 (between different layers).

In the second embodiment described above, the intermediate elastic bodies 65 are provided in the stretched state between the central elastic body 60 and the leakage preventive elastic bodies 51. Particularly, each of the intermediate elastic bodies 65 is provided in a position outward than a corresponding one of the side bent portions 43B in the width direction W of the absorbent article 1D. Thus, the pair of side bent portions 43B allow the absorber 40 to be more readily bent in a direction of separating from the wearer. As a result, the absorbent article 1 more readily fits the wearer. In other words, a so-called W-shape can be more surely formed.

In the second embodiment, it is preferable that the length (L6) of the intermediate elastic bodies 65 is larger than the length (L4) of the central crotch portion 41C. Note that, if the length (L6) of the intermediate elastic bodies 65 is smaller than the length (L4) of the central crotch portion 41C, the absorber 40 (the central crotch portion 41C) may not fit the wearer in a favorable manner.

### [Third Embodiment]

With reference to the drawings, a configuration of an absorbent article 1F according to a third embodiment will be described below. Note that the same parts as those of the absorbent article 1 according to the above-described first embodiment are denoted by the same reference numerals, and different parts will be mainly described.

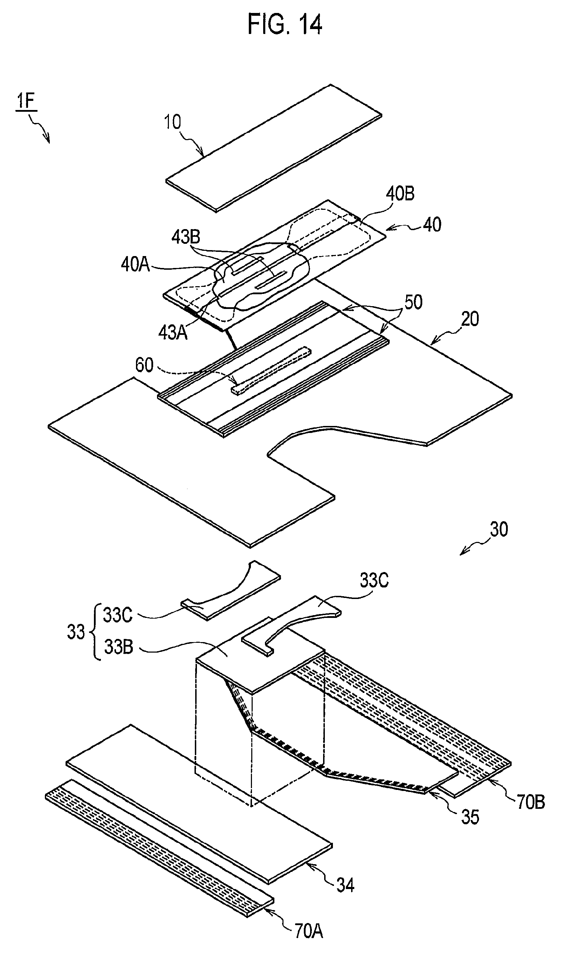

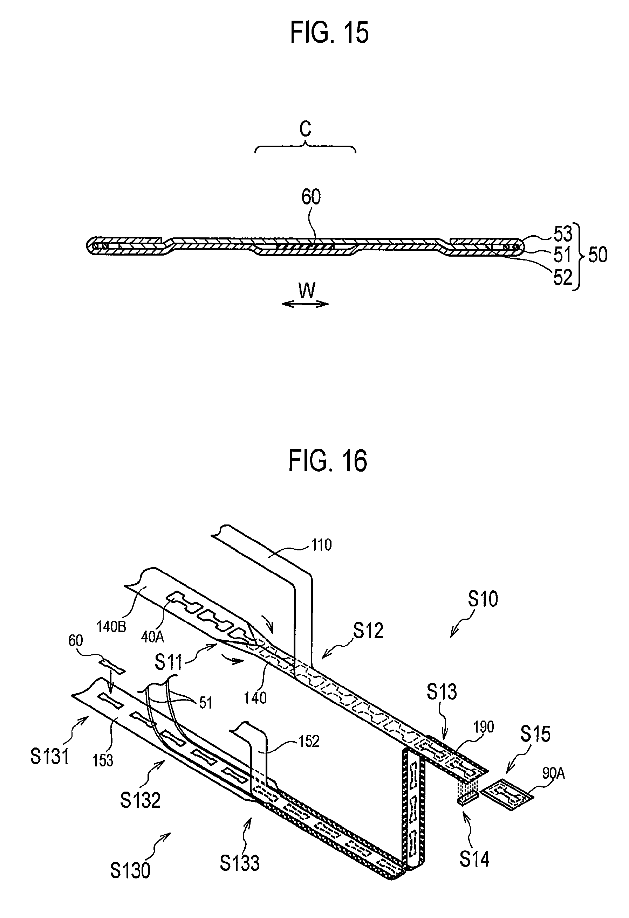

Fig. 14 is an exploded perspective view showing the absorbent article 1F according to the third embodiment. Fig. 15 is a cross-sectional view showing only the absorbent article 1F according to the third embodiment. In the absorbent article 1 according to the first embodiment described above, the central elastic body 60 is provided between the outer topsheet 20 and the outer sheet 30. Meanwhile, in the absorbent article 1F according to the third embodiment, a central elastic body 60 is provided between an outer topsheet 20 and an absorber 40.

To be more specific, as shown in Figs. 14 and 15, the central elastic body 60 is provided between a leakage preventive film sheet 52 and a leakage preventive nonwoven fabric sheet 53. Note that the central elastic body 60 is not necessarily provided between the leakage preventive film sheet 52 and the leakage preventive nonwoven fabric sheet 53, but may be provided, for example, between the outer topsheet 20 and the leakage preventive nonwoven fabric sheet 53, or may be provided between the absorber 40 and the leakage preventive film sheet 52.

In the third embodiment, a leakage preventive part 50 is continuously and integrally formed in the width direction W of the absorbent article 1F so as to be positioned at both ends 40E of the absorber 40. Moreover, in the absorbent article 1F according to the third embodiment, the front outer sheet 31, the rear outer sheet 32 and the crotch inner sheet 33A are not provided.

Next, with reference to the drawings, a description will be given of a method for manufacturing an absorbent article according to the third embodiment. Fig. 16 is a view for illustrating the method for manufacturing an absorbent article according to the third embodiment. Note that only steps different from those of the method for manufacturing an absorbent article according to the first embodiment will be described.

In the method for manufacturing an absorbent article according to the above-described first embodiment, after attaching the central elastic body 60 to the outer topsheet web 120, the absorber 40 is disposed so that the central bent portion 43A overlaps with the central elastic body 60. Meanwhile, in the method for manufacturing an absorbent article according to the third embodiment, the central elastic body 60 is disposed within the leakage preventive part 50 when forming the leakage preventive part 50.

To be more specific, as shown in Fig. 16, the method for manufacturing an absorbent article includes a leakage preventive part formation step S130. The leakage preventive part formation step S130 includes a central elastic body disposition step S131, a leakage preventive elastic body bonding step S132 and a leakage preventive film attachment step S133.

In the central elastic body disposition step S131, on a leakage preventive nonwoven fabric web 153 of the continuum of the leakage preventive nonwoven fabric sheets 53, the central elastic body 60 is disposed in a state of being stretched along a conveying direction MD of the leakage preventive nonwoven fabric web 153.

In the leakage preventive elastic body bonding step S132, on both sides of the leakage preventive nonwoven fabric web 153, leakage preventive elastic bodies 51 are bonded in a state of being stretched along the conveying direction MD of the leakage preventive nonwoven fabric web 153.

In the leakage preventive film attachment step S133, a leakage preventive film web 152 of the continuum of the leakage preventive film sheets 52 is attached to the leakage preventive nonwoven fabric web 153 on which the leakage preventive elastic bodies 51 and the central elastic body 60 are provided. Next, both edges of the leakage preventive nonwoven fabric web 153 are folded to wrap the leakage preventive elastic bodies 51 therein. Thus, a leakage preventive part web 150 of the continuum of the leakage preventive parts 50 is formed.

Note that, in the leakage preventive part attachment step S13 described above, the leakage preventive part web 150 is attached to the absorber web 140 so that at least a part of the central elastic body 61 overlaps with the central bent portion 43A.

Here, although the description has been given in the third embodiment for the case where the leakage preventive elastic body bonding step S132 is performed after the central elastic body disposition step S131, the present invention is not limited thereto. The central elastic body disposition step S131 and the leakage preventive elastic body bonding step S132 may otherwise be performed simultaneously. Alternatively, the central elastic body disposition step S131 may be performed after the leakage preventive elastic body bonding step S132.

In the third embodiment described above, the leakage preventive film web 152 is attached to the leakage preventive nonwoven fabric web 153 after the central elastic body 60 is disposed on the leakage preventive nonwoven fabric web 153. This means the central elastic body 60 is sandwiched between the leakage preventive nonwoven fabric web 153 and the leakage preventive film web 152. Accordingly, in conveying the web having the central elastic body 60 attached thereto, the central elastic body 60 sandwiched between the webs is less likely to contract compared with the case where the central elastic body 60 is not sandwiched between the webs. Thus, the manufacturing is facilitated so that the stretchability of the central elastic body 60 is not impaired but maintained. Therefore, the central elastic body 60 is not contracted during the manufacturing of the absorbent article 1F, and thus the central elastic body 60 can surely contract during use. As a result, the effect of causing the absorbent article 1 to attach to the skin of the wearer is more readily exerted.

### [Fourth Embodiment]

With reference to the drawings, a configuration of an absorbent article 1G according to a fourth embodiment will be described below. Note that the same parts as those of the absorbent article 1 according to the above-described first embodiment are denoted by the same reference numerals, and different parts will be mainly described.

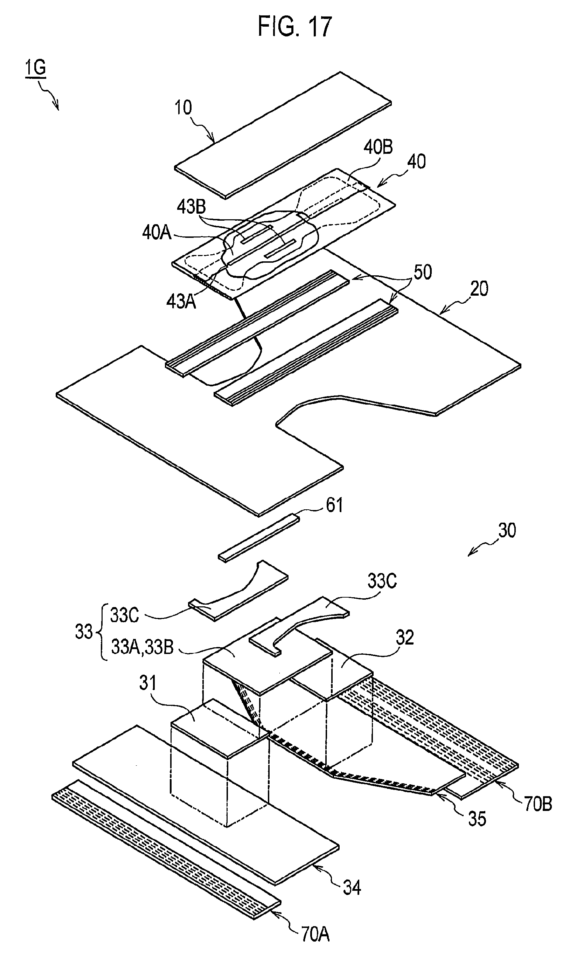

Fig. 17 is an exploded perspective view showing the absorbent article 1G according to the fourth embodiment. In the absorbent article 1 according to the first embodiment described above, the central elastic body 60 is a sheet such as a nonwoven fabric which is stretchable along the longitudinal direction L of the absorbent article 1. Meanwhile, in the absorbent article 1G according to the fourth embodiment, as shown in Fig. 17, a central elastic body 61 is synthetic rubber which is stretchable along a longitudinal direction L of the absorbent article 1G.

Note that a disposition position of the central elastic body 61 is the same as those of the central elastic bodies 60 described in the first to third embodiments. Moreover, as in the case of the central elastic body 61, the intermediate elastic bodies 65 described in the second embodiment may also be synthetic rubber which is stretchable along the longitudinal direction L of the absorbent article 1G, as a matter of course.

In the fourth embodiment described above, the central elastic body 61 is the synthetic rubber which is stretchable along the longitudinal direction L of the absorbent article 1G. Thus, compared with the central elastic body 60 formed of a sheet such as a nonwoven fabric as in the first embodiment, stress adjustment is more readily performed by changing the size, cross-sectional area (thickness), number, scale factor and the like of the central elastic body 61 (synthetic rubber) in accordance with the rigidity of the absorber 40. As a result, stretch stress of the central elastic body 61 can be properly and easily designed.

### (other Embodiments)

As described above, the contents of the present invention have been disclosed through the embodiments of the present invention. However, it should be understood that the present invention is not limited to the description and drawings which constitute a part of this disclosure. From this disclosure, various alternative embodiments, examples, and operation techniques will become apparent to those skilled in the art.

For example, the embodiments of the present invention may be modified as follows. Specifically, although the description has been given assuming that the absorbent article 1 is a shorts-type diaper, the present invention is not limited thereto and may be an open-type diaper, a sanitary napkin and the like.

Moreover, the members which constitute the absorbent article 1 (i.e. the topsheet 10, the outer topsheet 20, the outer sheet 30, the absorber 40, the leakage preventive part 50 and the central elastic body 60) are not limited to the configurations described in the embodiments, but may have different configurations and can be changed in accordance with the intended use. Similarly, the method for manufacturing an absorbent article can also be changed in accordance with the intended use, as a matter of course.

For example, a member (such as the leakage preventive part 50) may be omitted, or the absorber 40 may have a different shape (e.g., the connection parts 42A may be omitted). Furthermore, the front waistline outer sheet 34 and the front waist sheet 70A may be formed integrally, or the rear waistline outer sheet 35 and the rear waist sheet 70B may be formed integrally.

Note that, when the leg elastic bodies 7A which intersect with at least a part of the central elastic body 60 are provided as in the first embodiment, the front waistline outer sheet 34 and the rear waistline outer sheet 35 are not necessarily be formed of stretchable sheets but may be formed of non-stretchable sheets. Moreover, when the leg elastic bodies 7A are not provided, the front waistline outer sheet 34 or the rear waistline outer sheet 35 is preferably a stretchable sheet. In this case, it suffices that at least a part of the central elastic body 60 intersects with the stretchable sheet.

Moreover, although the description has been given assuming that the elastic bodies other than the central elastic body 60 are synthetic rubber which is stretchable along the longitudinal direction L of the absorbent article 1, the present invention is not limited thereto. A stretchable member such as natural rubber and polyurethane elastic fiber (so-called spandex) may be used, for example.

Moreover, although the description has been given assuming that the members constituting the absorbent article 1 are partially bonded to each other with an adhesive (e.g., hot-melt) or the like, the present invention is not limited thereto. The members may otherwise be bonded to each other by thermal fusion bonding or the like.

Moreover, although the description has been given assuming that, in the top-side joint, the topsheet 10 and the first crotch member 90A are bonded to each other, the present invention is not limited thereto. Any top-side sheet positioned closer to the topsheet 10 than the first crotch member 90A can be bonded to the first crotch member 90A. Similarly, also as to the outer-side joint, any outer-side sheet positioned closer to the outer sheet 30 than the first crotch member 90A can be bonded to the first crotch member 90A.

Moreover, although the description has been given assuming that the central elastic body 60 overlaps with the front waistline region S1 and the rear waistline region S2, the present invention is not limited thereto. It suffices that the central elastic body 60 overlaps with any one of the front waistline region S1 and the rear waistline region S2. Specifically, it suffices that the front end portion 60A overlaps with the front waistline region S1 or the rear end portion 60B overlaps with the rear waistline region S2.

Moreover, although the description has been given assuming that the bent part 43 has the central bent portion 43A and the side bent portions 43B, the present invention is not limited thereto, but the bent part 43 may be configured only of the central bent portion 43A. Moreover, the bent part 43 is not limited to the configuration described in the embodiments, but may have a different configuration and can be changed in accordance with the intended use.

Moreover, although the description has been given assuming that the central elastic body 60 overlaps with the central bent portion 43A in the thickness direction T of the absorbent article 1, the present invention is not limited thereto. It suffices that at least a part of the central elastic body 60 overlaps with the central bent portion 43A.

Moreover, although the description has been given assuming that the central elastic body 60 (the rear end portion 60B) intersects with the leg elastic bodies 7A, the present invention is not limited thereto. Alternatively, the central elastic body 60 may intersect not only with the leg elastic bodies 7A but also with the waist elastic bodies 5A. Note that the front end portion 60Amay intersect with the waist elastic bodies 3A.

Moreover, although the description has been given assuming that the bent part 43 has a slit shape penetrating the absorber 40, the present invention is not limited thereto. The bent part 43 need not penetrate the absorber 40 and may have the smallest thickness in the absorber 40. For example, the bent part 43 may be embossed to be thinly formed.

Moreover, although the description has been given assuming that the absorber 40 is formed of the powder mix 40A and the covering material 40B, the present invention is not limited thereto. For example, the absorber 40 may be formed of a sheet such as an airlaid nonwoven fabric. In this case, the bent part 43 is formed, for example, in the sheet such as the airlaid nonwoven fabric.

Hence, the present invention includes various embodiments and the like which are not described herein, as a matter of course. Therefore, a technological scope of the present invention is defined only by items specific to the invention according to claims pertinent based on the foregoing description.

Note that the entire contents of Japanese Patent Application No. 2009-136437 (filed on June 5, 2009) are incorporated herein by reference.

### [Industrial Applicability]

According to an aspect of the present invention, provided is an absorbent article more comfortably worn by a wearer while surely absorbing bodily waste, and a method for manufacturing the absorbent article.

## Claims

1. An absorbent article comprising:
a topsheet which comes into contact with the skin of a wearer;
an outer sheet positioned on an outer side during wearing;
an absorber provided between the topsheet and the outer sheet;
a front waistline region corresponding to a front waistline of the wearer;
a rear waistline region corresponding to a rear waistline of the wearer;
a first elastic body provided to be stretchable along a width direction of the absorbent article; and
a second elastic body provided to be stretchable along a longitudinal direction of the absorbent article, wherein
the front waistline region and the rear waistline region each has a pair of edges positioned at outer ends in the width direction of the absorbent article,
in the absorber, a bent part is provided, which is formed along the longitudinal direction of the absorbent article,
the first elastic body continuously extends from one edge to the other edge in at least one of the front waistline region and the rear waistline region,
the second elastic body is provided in a central portion in the width direction of the absorbent article in a state of being stretched in the longitudinal direction of the absorbent article, and overlaps with any one of the front waistline region and the rear waistline region in a thickness direction of the absorbent article, and
at least a part of the second elastic body intersects with the first elastic body while overlapping with the bent part in the thickness direction of the absorbent article.

2. The absorbent article according to claim 1, wherein
a length of the second elastic body along the longitudinal direction of the absorbent article is smaller than a length of the bent part along the longitudinal direction of the absorbent article.

3. The absorbent article according to any one of claims 1 and 2, further comprising:
a third elastic body provided to be stretchable along the longitudinal direction of the absorbent article, wherein
the third elastic body is provided between the second elastic body and each of both sides of the absorber in a state of being stretched in the longitudinal direction of the absorbent article.

4. The absorbent article according to any one of claims 1 to 3, wherein the bent part includes
a central bent portion formed in a central portion in the width direction of the absorbent article, and
side bent portions formed on outer sides of the central bent portion in the width direction of the absorbent article.

5. The absorbent article according to any one of claims 1 to 4, further comprising:
a top-side joint in which a top-side sheet positioned closer to the topsheet than the absorber is bonded to the absorber; and
an outer-side joint in which an outer-side sheet positioned closer to the outer sheet than the absorber is bonded to the absorber;
wherein the second elastic body is provided so as to be shifted from the top-side joint or the outer-side joint in the width direction of the absorbent article.

6. The absorbent article according to any one of claims 1 to 5, wherein
a crotch region corresponding to the crotch of the wearer is provided between the pair of waistline regions,
the absorber includes
a waistline portion positioned in the front waistline region and the rear waistline region,
a central crotch portion positioned in the center of the crotch region in the longitudinal direction of the absorbent article, and
an intermediate crotch portion positioned between the waistline portion and the central crotch portion, and
a width of the intermediate crotch portion along the width direction of the absorbent article is smaller than a width of the central crotch portion along the width direction of the absorbent article.

7. The absorbent article according to claim 6, further comprising:
a third elastic body which is provided along the longitudinal direction of the absorbent article and is stretchable along the longitudinal direction of the absorbent article, wherein
the third elastic body is provided between the second elastic body and each of both sides of the absorber in a state of being stretched in the longitudinal direction of the absorbent article, while overlapping with the central crotch portion in the thickness direction of the absorbent article.

8. The absorbent article according to any one of claims 6 and 7, further comprising:
a third elastic body which is provided along the longitudinal direction of the absorbent article and is stretchable along the longitudinal direction of the absorbent article, wherein
a length of the third elastic body along the longitudinal direction of the absorbent article is larger than a length of the central crotch portion along the longitudinal direction of the absorbent article.

9. A method for manufacturing an absorbent article including a liquid-permeable topsheet which comes into contact with the skin of a wearer, an outer sheet positioned on an outer side during wearing, an absorber provided between the topsheet and the outer sheet, a front waistline region corresponding to a front waistline of the wearer, and a rear waistline region corresponding to a rear waistline of the wearer, the method comprising the steps of:
bonding at least a part of a first elastic body in a stretched state to at least one of a front waistline web of the continuum of the front waistline regions and a rear waistline web of the continuum of the rear waistline regions, the first elastic body being stretchable along a conveying direction of the front waistline web and the rear waistline web;
disposing a second elastic body in a stretched state between the front waistline web and the rear waistline web, the second elastic body being stretchable along an orthogonal direction perpendicular to the conveying direction of the front waistline web and the rear waistline web;
disposing the absorber between the front waistline web and the rear waistline web, the absorber having a bent part formed along the longitudinal direction of the absorbent article, wherein
in the step of disposing the second elastic body in a stretched state, the second elastic body is caused to overlap with any one of the front waistline web and the rear waistline web in the thickness direction of the absorbent article, while at least a part of the second elastic body is caused to overlap with the first elastic body, and
in the step of disposing the absorber, the bent part is caused to overlap with at least a part of the second elastic body.

10. A method for manufacturing an absorbent article including a liquid-permeable topsheet which comes into contact with the skin of a wearer, an outer sheet positioned on an outer side during wearing, an absorber provided between the topsheet and the outer sheet, a front waistline region corresponding to a front waistline of the wearer, and a rear waistline region corresponding to a rear waistline of the wearer, the method comprising the steps of:
forming the absorber having a bent part formed therein along a longitudinal direction of the absorbent article;
attaching at least a topsheet web of the continuum of the topsheets to the absorber;
bonding at least a part of a first elastic body in a stretched state to at least one of a front waistline web of the continuum of the front waistline regions and a rear waistline web of the continuum of the rear waistline regions, the first elastic body being stretchable along a conveying direction of the front waistline web and the rear waistline web; and
disposing the absorber between the front waistline web and the rear waistline web, the absorber having a bent part formed along the longitudinal direction of the absorbent article, wherein
in the step of attaching the topsheet web, a second elastic body which is stretchable along a conveying direction of the topsheet web is disposed in a stretched state, while the second elastic body is attached so as to overlap with at least a part of the bent part, and
in the step of disposing the absorber, the second elastic body is caused to overlap with any one of the front waistline web and the rear waistline web in the thickness direction of the absorbent article, while at least a part of the second elastic body is caused to overlap with the first elastic body.
